## Europäisches Patentamt
(19) **European Patent Office**

**Office européen des brevets**

(11) Publication number: **0 011 658**
B1

(12) **EUROPEAN PATENT SPECIFICATION**

(45) Date of publication of patent specification: **19.05.82**

(51) Int. Cl.³: **C 23 G 5/02, C 07 C 17/42**

(21) Application number: **78101510.2**

(22) Date of filing: **01.12.78**

(54) **Stabilized methylene chloride formulation for vapor degreasing.**

(43) Date of publication of application:
**11.06.80 Bulletin 80/12**

(45) Publication of the grant of the patent:
**19.05.82 Bulletin 82/20**

(84) Designated Contracting States:
**BE DE FR GB IT NL SE**

(56) References cited:
**US - A - 2 818 446**
**US - A - 3 002 028**

(73) Proprietor: **DOW CHEMICAL (EUROPE) S.A.**
**Bachtobelstrasse 3**
**CH-8810 Horgen (CH)**

(72) Inventor: **Sehr, Wolf Juergen**
**Sennhüttenstrasse 19**
**CH-8810 Horgen (CH)**
Inventor: **Scheu, Andreas Karl Heinrich**
**Brandschenkestrasse 38**
**CH-8002 Zürich (CH)**

(74) Representative: **Casalonga, Alain et al,**
**BUREAU D.A. CASALONGA OFFICE JOSSE &**
**PETIT Baaderstrasse 12-14**
**D-8000 München 5 (DE)**

### Stabilized methylene chloride formulation for vapor degreasing

Methylene chloride is a highly versatile and useful solvent for various industrial applications at both normal and elevated temperatures. A particularly important industrial use is the degreasing of metals which is widely practiced today. Numerous solvents have been employed, and numerous variations of the degreasing procedure have been suggested. In one method of operating, the metal article to be degreased is brought into contact with the solvent in the liquid phase. This may be accomplished by immersing the article in a large body of the solvent, or by spraying the solvent on the surface of the article. In another common method of degreasing, known generally as vapor degreasing, the solvent is maintained at the boiling point and in communication with a chamber adapted to contain a large amount of the solvent vapor. The article to be degreased is brought into contact with this vapor, and the solvent then condenses on the greasy metal surface. The condensed solvent removes grease and oil from the metal surface and drips off, usually returning to the boiling solvent. Along with it goes dirt, which had adhered to the greasy surface. This dirt often includes metal chips.

Methylene chloride is known to be more stable than other chlorinated hydrocarbon solvents such as perchloroethylene, trichloroethylene and methyl chloroform when the solvents are used in unstabilized condition. For example, methylene chloride is more resistant to oxidation, hydrolysis and pyrolysis than other chlorinated solvents and does not substantially react with aluminum in the aluminum scratch test commonly used to indicate unstabilized or minimally stabilized methyl chloroform. In addition, methylene chloride may be used to greater advantage in vapor degreasing of metals than other known degreasing solvents, since it can be used effectively at lower temperatures due to its lower boiling point and excellent stability. Methylene chloride is particularly desirable for such degreasing operations since it is substantially resistant to photochemical activity and therefor does not contribute to air pollution by smog formation. However, methylene chloride when used in various metal cleaning functions, including vapor degreasing, suffers the disadvantage of being reactive with aromatic compounds in the presence of metals, metal halides and combinations thereof. The presence of the said metals and halides causes a reaction with the methylene chloride to produce hydrochloric acid and objectionable high boiling tarry substances which render the methylene chloride unsuitable for further use. The entire reaction of methylene chloride in the presence of aromatic compounds is not completely understood but is believed to be a condensation reaction catalyzed by metal, metal halides or combinations thereof. Thus, the aromatic compound reaction with methylene chloride is believed to be catalyzed, or initiated and catalyzed by the presence of metals such as aluminum, zinc, iron and the like, halides of said metals and combinations of said metals and halides. Such reactions are thought to be of the nature of a Friedel-Craft type reaction.

Aromatic compounds and metals such as aluminum, iron and zinc, their halides and combinations are generally introduced into the methylene chloride from various cutting oils and lubricants used in metal fabricating operations which are carried over into the methylene chloride solvent during vapor degreasing or other cleaning of the fabricated metal parts. Solvent manufacturing, handling and storage equipment is another source for introduction of such impurities. To prevent degradation and other types of deterioration such as oxidation, hydrolysis and pyrolysis which may occur in some instances, it has been the practice to incorporate minor quantities of various organic compounds into methylene chloride, which compounds act as stabilizers to substantially prevent such degradation. It is desirable to provide methylene chloride stabilized to effectively prevent degradation in various applications, and the demand still exists for a low cost stabilized methylene chloride composition that can be easily prepared and which provides optimum stabilization under many different operating conditions.

It is particularly desirable that the inhibitor be present in the vapor as well as the liquid phase since, if the vapor condenses in the absence of inhibitor, the potential for degrading the solvent is created when the condensate contacts the metal parts containing the cutting oils. The metal chips or cuttings also find their way into the boiling bath of solvent so that the inhibitor must also be there.

Representative stabilizer compounds employed in chlorinated hydrocarbons used for degreasing metals are those employed in trichloroethylene. Thus, cyclic sulfones, pyrrol or N-alkyl pyrroles, phenol and an epoxide, e.g. butylene oxide, and carboxylic acid esters, e.g. ethyl acetate (U.S.—A—2,371,647) among others, have all been employed. Various other amines, ethers, thio compounds and diolefins have also been used alone or in combination with other components as stabilizers for, trichloroethylene. Dioxane, a tertiary acetylenic alcohol and a volatile nitrogen organic compound have been employed also as components of a stabilizer for trichloroethylene.

U.S.—A—2,818,446 discloses the use of a synergistically cooperative pair of additives consisting of an epoxide and an ester for stabilizing trichlorethylene. The association disclosed in the working examples and used in conditions simulating vapor degreasing, comprise. components having boiling points very different from that of the solvent.

Not all of these stabilizers are effective to protect the chlorinated solvents against decomposition in the presence of metals. Some are only effective against oxidative and thermal decomposition in the presence of heat, light and air.

Although not employed as a degreasing solvent in U.S.—A—3,646,229, methylene chloride is stabilized against the action of aluminum in the presence of aromatic hydrocarbons in a paint composition by empolying dimethoxy methane as a stabilizer.

U.S.—A—2,371,647 teaches the use of many different carboxylic acid esters as stabilizers for various saturated and unsaturated chlorinated hydrocarbons. Thus, for example, ethyl acetate, dimethyl carbonate, dimethyl oxalate, n-butyl formate, ethyl benzoate and n-butyl butyrate are suggested as stabilizers. Preferred are the esters derived from alcohols and acids of five or fewer carbon atoms. A more recent patent (U.S.—A—3,864,408) employs methyl acetate alone or in combination with propylene oxide as a stabilizer for methylene chloride.

There has now been found, according to the present invention, an especially good stabilizer for methylene chloride, which has the necessary volatility to be carried into the vapor phase and thus be present in the condensate when used in vapor degreasing operations, and which has an especially good stabilizing effect for methylene chloride in the presence of aromatic solvents and aluminum metal.

Accordingly the present invention is a stabilized solvent composition suitable for vapor degreasing of aluminum parts which comprises methylene chloride and characterized in that the stabilizer comprises methyl and/or ethyl formate. In another aspect, the present invention is a stabilized solvent composition suitable for vapor degreasing of aluminum parts which comprises methylene chloride and further characterized in that the stabilizer is a mixture of methyl formate and propylene oxide.

Methyl formate may be employed alone when used in a concentration of at least about 1.5 percent by volume. When employed in combination with ethyl formate or propylene oxide, the methyl formate and the second component are used in approximately equal volumes which together amount to at least about 1 percent by volume, and preferably not more than about 2 percent by volume, based on the methylene chloride. Ethyl formate may be employed alone at a concentration of at least about 2 percent by volume in the methylene chloride. Normally, these inhibitor systems will be used in a concentration of not more than 5 percent by volume, and preferably not more than 3 percent by volume to obtain the desired protection. Excessive amounts of inhibitor do not provide additional protection, and thus are wasteful of inhibitor.

The present invention is also directed to a process for vapor degreasing of aluminum parts by employing methylene chloride with the amounts of stabilizers described above.

The stabilizers of the invention were tested according to procedures set forth by the German BAM (Bundesanstalt fur Materialprüfung).

One test involved mixing equal volumes of stabilized solvent and toluene and placing therein aluminum metal flakes and a small amount of aluminum chloride crystals. The mixture is heated for 18 hours at reflux temperature under atmospheric pressure. Another test under the same conditions employed a small amount of zinc stearate and yet another employed oleic acid in like manner. The above tests are employed in Example 2.

### Example 1

The following stabilizers were tested in methylene chloride by employing 100 cc of the stabilized solvent and 100 cc toluene containing 18 g of flake aluminum metal and 0.7 g of aluminum chloride crystals.

| Compound | Concentration (Volume Percent) |
|---|---|
| 1. Methyl formate | 1 |
| 2. Methyl formate | 1.5 |
| 3. Ethyl formate | 1 |
| 4. Ethyl formate | 2 |
| 5. Methyl formate<br>Ethyl formate | 0.5<br>0.5 |
| 6. Methyl formate<br>Ethyl formate | 0.7<br>0.7 |

The above six formulations showed no exotherm during an 18-hour reflux period.

In like manner, 1 g of zinc stearate was refluxed for 18 hours with 100 cc each of the above six formulations and 100 cc of toluene, with no evidence of an exotherm. Similarly, 10 cc of oleic acid was refluxed with the same amounts of stabilized methylene chloride and toluene for each of the above six formulations with no evidence of an exotherm for the test period.

### Examples 2—5

Other samples of the same six formulations used in Example 1 were fractionated into three fractions of 100 cc each. Fractions 1, 2 and 3 designate the first and second fractions taken overhead and the remainder in the flask, respectively. The reflux test was then performed with toluene and aluminum metal and aluminum chloride as in Example 1 for each of the fractions. The time to exotherm (if any) is shown. A "yes" indicates that the fraction is sufficiently stabilized, i.e. enough of the inhibitor was carried over in the distillation to be effective for the test period. Results are shown also for inhibitors known to the art and are designated as Comparative Examples. The results for all tests are given in the Table following.

TABLE

| Stabilizers and Amount (Volume Percent)* | | Fraction 1 (hours) | Fraction 2 (hours) | Fraction 3 (hours) | Test Passed |
|---|---|---|---|---|---|
| Comparative A — Dimethoxymethane | (1) | <6 | >18 | <18 | no |
| Comparative B — Methyl acetate | (1) | <1 | >18 | >18 | no |
| Comparative C — Propylene oxide | (1) | <1 | <2 | >18 | no |
| Comparative D — Dioxane | (1) | <1 | <1 | >18 | no |
| Comparative E — Methyl alcohol | (1) | <1 | <1 | <9 | no |
| Comparative F — Methyl formate | (1) | >18 | >18 | <4 | no |
| Example 2 — Methyl formate | (1.5) | >18 | >18 | >18 | yes |
| Comparative G — Ethyl formate | (1) | <1 | <2 | >18 | no |
| Example 3 — Ethyl formate | (2) | >18 | >18 | >18 | yes |
| Example 4 — Methyl formate + Ethyl formate | (0.5) (0.5) | >18 | >18 | >18 | yes |
| Example 5 — Methyl formate + Propylene oxide | (0.7) (0.7) | >18 | >18 | >18 | yes |

* The amount of stabilizer is that charged to the original 300 cc of solvent prior to distillation.

Claims

1. A stabilized solvent composition suitable for vapor degreasing of aluminum parts which comprises methylene chloride and characterized in that the stabilizer comprises methyl and/or ethyl formate.

2. A stabilized solvent composition as claimed in Claim 1 and further characterized in that the stabilizer is a mixture of methyl formate and propylene oxide.

3. The composition of Claim 1 further characterized in that methyl formate is present at a concentration of at least 1.5 percent by volume.

4. The composition of Claim 1 further characterized in that ethyl formate is present at a concentration of at least 2 percent by volume.

5. The composition of Claim 1 further characterized in that methyl and ethyl formate are each present in a concentration of at least 0.5 percent each by volume.

6. The composition of Claim 2 further characterized in that the methyl formate and propylene oxide are present in a concentration of at least 0.7 percent each by volume.

Revendications

1. Composition de solvant stabilisée appropriée pour le dégraissage en phase vapeur de particules d'aluminium qui comprend du chlorure de méthylène, caractérisée par le fait que le stabilisant comprend du forimate de méthyle et/ou d'éthyle.

2. Composition de solvant stabilisé telle que définie dans la revendication 1, caractérisée encore par le fait que le stabilisant est un mélange de forimate de méthyle et d'oxyde de propylène.

3. Composition selon la revendication 1, caractérisée encore par le fait que le forimate de méthyle est présent à une concentration d'au moins 1,5% en volume.

4. Composition selon la revendication 1, caractérisée encore par le fait que le forimate d'éthyle est présent à une concentration d'au moins 2% en volume.

5. Composition selon la revendication 1, caractérisée encore par le fait que le forimate de méthyle et d'éthyle sont présents chacun dans une concentration d'au moins 0,5% en volume pour chacun.

6. Composition selon la revendication 2, caractérisée encore par le fait que le forimate de méthyle et l'oxyde de propylène sont présents dans des concentrations d'au moins 0,7% chacun par volume.

**Patentansprüche**

1. Eine stabilisierte Lösungsmittelzusammensetzung, geeignet zur Dampfentfettung von Aluminiumteilen, die Methylenchlorid (Dichlormethan) enthält, dadurch gekennzeichnet, dass das Stabilisierungsmittel Methyl- und/oder Ethylformiat enthält.

2. Eine stabilisierte Lösungsmittelzusammensetzung gemäss Anspruch 1, ferner dadurch gekennzeichnet, dass das Stabilisierungsmittel aus einem Gemisch von Methylformiat und Propylenoxid besteht.

3. Die Zusammensetzung gemäss Anspruch 1, ferner dadurch gekennzeichnet, dass das Methylformiat in einer Konzentration von mindestens 1,5 Volumenprozent vorliegt.

4. Die Zusammensetzung gemäs Anspruch 1, ferner dadurch gekennzeichnet, dass das Ethylformiat in einer Konzentration von mindestens 2 Volumenprozent vorliegt.

5. Die Zusammensetzung gemäss Anspruch 1, ferner dadurch gekennzeichnet, dass Methyl- und Ethylformiat jeweils in einer Konzentration von 0,5 Volumenprozent vorliegen.

6. Die Zusammensetzung gemäss Anspruch 2, ferner dadurch gekennzeichnet, dass das Methylformiat und das Propylenoxid in einer Konzentration von jeweils mindestens 0,7 Volumenprozent vorliegen.